# EUROPEAN PATENT APPLICATION

(11) **EP 2 913 401 A2**
(43) Date of publication of application: **02.09.2015**
(21) Application number: 15156290.7
(22) Date of filing: 24.02.2015
(51) Int. Cl.: C12N 15/10

(54) **Lyophilizate of substance-binding solid-phase carrier, vessel for binding substance in substance-containing liquid to substance-binding solid-phase carrier, and method of producing lyophilizate containing substance-binding solid-phase carrier**

(30) Priority: 26.02.2014 JP 2014035262
(71) Applicant: Seiko Epson Corporation, Shinjuku-ku Tokyo 163-0811 (JP)
(72) Inventor: Koeda, Hiroshi, Nagano, 392-8502 (JP); Idegami, Kotaro, Nagano, 392-8502 (JP)
(74) Representative: Hoffmann Eitle

(57) **Abstract**

A lyophilizate containing a substance-binding solid-phase carrier, which is obtained by lyophilizing the substance-binding solid-phase carrier together with an additive, a vessel for binding a substance in a substance-containing liquid to a substance-binding solid-phase carrier, in which a lyophilizate obtained by lyophilizing the substance-binding solid-phase carrier together with an additive is provided, and a method of producing a lyophilizate containing a substance-binding solid-phase carrier, which includes lyophilizing the substance-binding solid-phase carrier together with an additive, are provided.

## Description

### BACKGROUND

### 1. Technical Field

The present invention relates to a lyophilizate of a substance-binding solid-phase carrier, a vessel for binding a substance in a substance-containing liquid to a substance-binding solid-phase carrier, and a method of producing a lyophilizate containing a substance-binding solid-phase carrier.

### 2. Related Art

In recent years, as a result of development of technologies utilizing genes, medical treatments utilizing genes such as gene diagnosis or gene therapy are drawing attention. In addition, many methods utilizing genes in determination of breed varieties or breed improvement have also been developed in agricultural and livestock industries. As technologies for utilizing genes, PCR (Polymerase Chain Reaction) and the like are widely used. Nowadays, PCR has become an indispensable technology for elucidation of information on biological materials. PCR is a method of amplifying a target nucleic acid by subjecting a solution (a reaction mixture) containing a nucleic acid to be amplified (a target nucleic acid) and a reagent to a thermal cycle. In the thermal cycle of PCR, a method of performing a thermal cycle in two or three temperature stages is generally used.

On the other hand, the diagnosis of an infectious disease as represented by influenza in medical practice is currently carried out mainly by using a simple test kit such as an immunochromatograph. However, such a simple test sometimes provides insufficient accuracy, and the application of PCR, which can be expected to provide higher test accuracy, to the diagnosis of an infectious disease has been eagerly awaited. Further, in general outpatient practice or the like in a medical institution, because the consultation time is limited, the time that can be spent for testing is limited to a short period. Due to this, the current situation is that, for example, the diagnosis of influenza is carried out by a simple test using an immunochromatograph or the like in a shorter time at the sacrifice of test accuracy. In light of such circumstances, in order to realize a test using PCR, which can be expected to provide higher test accuracy, in medical practice, it was necessary to reduce a time required for the reaction.

As a method of extracting a nucleic acid in a short time before a PCR reaction is performed, JP-A-2009-207459 discloses a method in which a nucleic acid is adhered to microbeads having magnetism and an operation is performed using a magnet.

A nucleic acid-binding solid-phase carrier (such as magnetic particles) is generally aggregated by its magnetism or the like when it is left to stand. Therefore, immediately before it is used for PCR or the like, it is necessary to disperse the nucleic acid-binding solid-phase carrier in a liquid.

### SUMMARY

An advantage of some aspects of the invention is to provide a vessel for binding a substance to a substance-binding solid-phase carrier in a shorter time by omitting an operation for dispersing the substance-binding solid-phase carrier.

A lyophilizate of a substance-binding solid-phase carrier according to an aspect of the invention is configured such that a substance-binding solid-phase carrier is lyophilized together with an additive. The substance-binding solid-phase carrier is preferably a magnetic particle. The additive may be trehalose, sucrose, polyvinylpyrrolidone, carboxymethyl cellulose, dextrin, dextran, polyethylene glycol, cyclodextrin, or maltodextrin.

A vessel for binding a substance in a substance-containing liquid to a substance-binding solid-phase carrier according to another aspect of the invention is configured such that a lyophilizate obtained by lyophilizing the substance-binding solid-phase carrier together with an additive is provided in the vessel. The vessel may include an opening section for introducing the substance-containing liquid; a filter section for filtering the substance-containing liquid introduced from the opening section; a contact section for bringing the substance-containing liquid passing through the filter section and the lyophilizate into contact with each other; and an outflow section for allowing the substance-containing liquid brought into contact with the lyophilizate in the contact section to flow out to the outside of the vessel.

A method of producing a lyophilizate containing a substance-binding solid-phase carrier according to still another aspect of the invention includes lyophilizing the substance-binding solid-phase carrier together with an additive. The substance-binding solid-phase carrier is preferably a magnetic particle. The additive may be trehalose, sucrose, polyvinylpyrrolidone, carboxymethyl cellulose, dextrin, dextran, polyethylene glycol, cyclodextrin, or maltodextrin.

### BRIEF DESCRIPTION OF THE DRAWINGS

The invention will be described with reference to the accompanying drawings, wherein like numbers reference like elements.
FIG. 1 is a schematic cross-sectional view showing a vessel according to an embodiment.
FIG. 2 is a cross-sectional view schematically showing the cross section of the vessel according to the embodiment taken along the line A-A in FIG. 1.
FIG. 3 is a cross-sectional view schematically showing the cross section of the vessel according to the embodiment taken along the line B-B in FIG. 1.
FIGS. 4A to 4E are schematic views showing a nucleic acid extraction method using the vessel according to the embodiment.
FIGS. 5A to 5E are schematic views showing a protein purification method using the vessel according to the embodiment.

### DESCRIPTION OF EXEMPLARY EMBODIMENTS

Hereinafter, embodiments of the invention will be described. The embodiments described below are mere examples of the invention. The invention is by no means limited to the embodiments described below and also encompasses various modifications carried out within the scope in which the gist of the invention is not changed. Incidentally, not all structures described below are necessarily essential components of the invention.

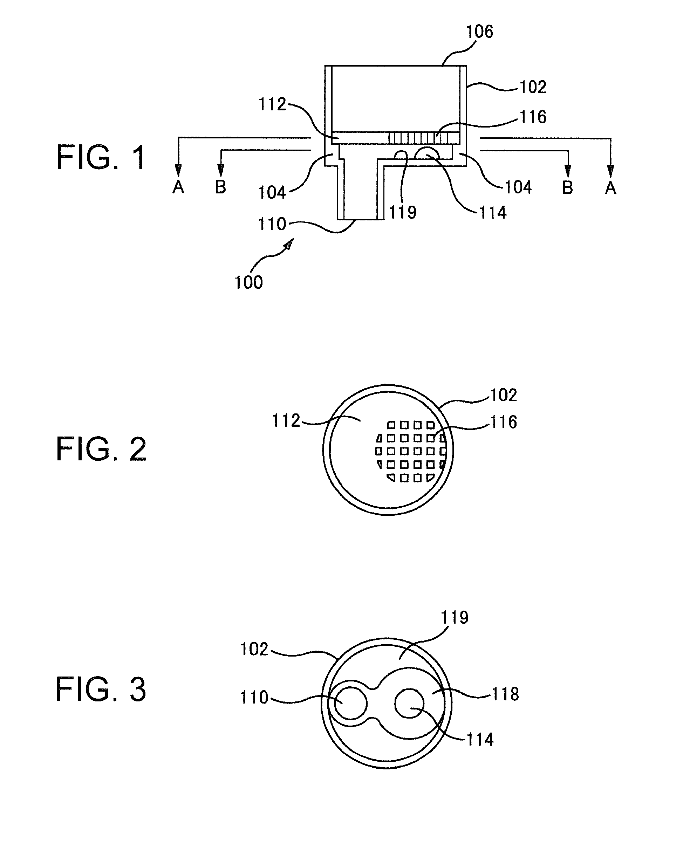

FIG. 1 is a schematic cross-sectional view of a vessel for bringing a substance in a substance-containing liquid and a substance-binding solid-phase carrier into contact with each other according to this embodiment. The "substance" as used herein refers to, for example, a nucleic acid such as DNA or RNA, a protein, or the like.

A vessel 100 according to the invention includes a filter installation section 112, a lyophilizate 114, a concave section 118, and an outlet port 110. The filter installation section 112 is, for example, a plastic plate with holes each having a diameter of several millimeters or a side of several millimeters. Preferably, a two-layer filter including a fine-mesh filter and a coarse-mesh filter is installed in the filter installation section 112.

The fine-mesh filter is installed downstream of the coarse-mesh filter. The fine-mesh filter and the coarse-mesh filter may be any as long as they have pores through which the substance can pass. For example, the size of the pores of the coarse-mesh filter can be set to 50 to 200 µm, and the size of the pores of the fine-mesh filter can be set to 10 to 30 µm. The material of the coarse-mesh filter and the fine-mesh filter is not particularly limited, however, for example, they can be prepared using polypropylene, polyethylene, polystyrene, nitrocellulose, or glass fiber. In this embodiment, in order to prevent the filter from clogging up, the filter is composed of two layers including the coarse-mesh filter and the fine-mesh filter, but may be composed only of one layer. Further, the filter may not be provided depending on the amount of contaminants in the substance-containing liquid.

When the substance-containing liquid is introduced into the vessel 100 from an opening section 106, the substance-containing liquid is filtered through the two-layer filter, whereby residues such as cells or viruses are removed from the substance-containing liquid. The substance-containing liquid passing through the pores of the filter comes in contact with the lyophilizate 114 to dissolve the lyophilizate. Then, the substance in the substance-containing liquid is bound to the substance-binding solid-phase carrier in the lyophilizate 114. From the thus obtained carrier having the substance bound thereto, the substance can be isolated according to, for example, a substance extraction method as described below. The material of the vessel 100 is not particularly limited, and a plastic made of a polymer, a metal, or the like can be used. FIG. 2 schematically shows the cross section of the vessel taken along the line A-A in FIG. 1. As shown in FIG. 2, it is preferred that holes 116 of the filter installation section 112 are provided in a portion of the surface so that the substance-containing liquid passing through the holes 116 flows to the lyophilizate 114. However, the holes of the filter installation section 112 may be provided on the entire surface, and a plastic sheet or the like with holes 116 in a portion thereof is interposed between the filter installation section 112 and the filter, so that the substance-containing liquid flows to the lyophilizate 114.

The lyophilizate 114 is a material obtained by lyophilizing an additive and a substance-binding solid-phase carrier, and is prepared by dispersing the substance-binding solid-phase carrier in a liquid to which the additive is added, followed by lyophilization while keeping the substance-binding solid-phase carrier in a dispersed state. In this lyophilizate 114, the substance-binding solid-phase carrier is present in a dispersed state. The additive to be lyophilized together with the substance-binding solid-phase carrier may be any as long as it is a substance which can keep the substance-binding solid-phase carrier in a dispersed state after lyophilization and is easily dissolved when it is brought into contact with water, and for example, trehalose, sucrose, polyvinylpyrrolidone, carboxymethyl cellulose, dextrin, dextran, polyethylene glycol, cyclodextrin, maltodextrin, or the like can be used. The addition amount of the additive to be lyophilized together with the substance-binding solid-phase carrier in the liquid may be any as long as the amount can keep the substance-binding solid-phase carrier in a dispersed state after lyophilization, and for example, it is preferred to add trehalose in an amount of 200 to 2000 µg per 10 µL of the liquid. The substance-binding solid-phase carrier may be any as long as it can bind the substance on the surface thereof, and for example, a known substance such as silica particles, polymer particles, or magnetic particles can be used, however, it is preferred to use magnetic particles.

FIG. 3 is a cross-sectional view schematically showing the cross section of the vessel according to the embodiment taken along the line B-B in FIG. 1. The concave section 118 is a part in which the substance-containing liquid is brought into contact with the lyophilizate 114. The concave section 118 is a part in the form of a basin with a bottom surface 119 of the vessel 100. To the concave section 118, the lyophilizate 114 is adhered, and also the outlet port 110 is formed therein. The substance-containing liquid passing through the filter flows along the concave section 118 on the bottom surface 119 and flows out to the outside of the vessel through the outlet port 110.

### Nucleic Acid Extraction Method

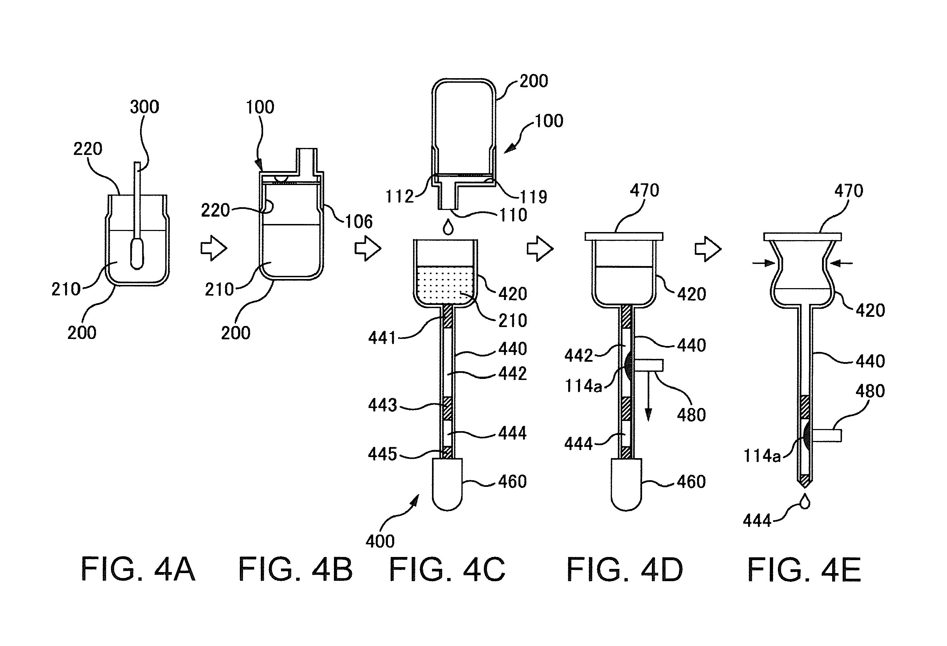

Next, a method of extracting a nucleic acid using the vessel 100 according to this embodiment will be described with reference to FIGS. 4A to 4E. FIGS. 4A to 4E are schematic views showing the nucleic acid extraction method using the vessel according to the embodiment.

First, the vessel 100, a bottle 200, and a nucleic acid extraction device 400 to be used in the nucleic acid extraction method will be described.

An upper portion of the bottle 200 has an opening section 220. In this embodiment, the opening section 220 of the bottle 200 is configured to be capable of being fitted with the opening section 106 of the vessel 100, and by fitting these opening sections together, the inside of the bottle 200 and the inside of the vessel 100 can communicate with each other.

The internal volume of the bottle 200 is not particularly limited, but can be set to, for example, 0.1 mL or more and 100 mL or less. The material of the bottle 200 is not particularly limited, and a plastic, a metal, or the like can be used.

A liquid is placed in the bottle 200. The liquid is not particularly limited as long as it contains a chaotropic substance, but may contain a surfactant for the purpose of disrupting cell membranes or denaturing proteins contained in cells. This surfactant is not particularly limited as long as it is generally used for extracting nucleic acids from cells or the like. Specific examples thereof include nonionic surfactants such as Triton surfactants (such as Triton-X) and Tween surfactants (such as Tween 20) and anionic surfactants such as sodium N-lauroylsarcosinate (SLS). However, particularly, it is preferred to use a nonionic surfactant in an amount ranging from 0.1 to 2%. Further, the liquid preferably contains a reducing agent such as 2-mercaptoethanol or dithiothreitol. The liquid may be a buffer, but preferably has a neutral pH ranging from 6 to 8. In view of this, specifically, the liquid preferably contains a guanidine salt (3 to 7 M), a nonionic surfactant (0 to 5%), EDTA (0 to 0.2 mM), a reducing agent (0 to 0.2 M), etc.

The chaotropic substance is not particularly limited as long as it generates a chaotropic ion (a monovalent anion having a large ionic radius) in an aqueous solution, has an activity to increase the water solubility of a hydrophobic molecule, and contributes to the adsorption of a nucleic acid on the fine particles. Specific examples thereof include guanidine thiocyanate, guanidine hydrochloride, sodium iodide, potassium iodide, and sodium perchlorate. Among these, guanidine thiocyanate or guanidine hydrochloride having a high protein denaturation activity is preferred. The concentration of such a chaotropic substance varies depending on the respective substances, and for example, when guanidine thiocyanate is used, the concentration thereof is preferably in the range of 3 to 5.5 M, and when guanidine hydrochloride is used, the concentration thereof is preferably 5 M or more.

The liquid preferably contains an alcohol or acetonitrile. In this case, the concentration of the alcohol or the like is not particularly limited, however, the lower limit thereof may be 10% or more, or may be 20% or more, or may be 30% or more, but most preferably 40% or more. The upper limit thereof may be 80% or less, or may be 70% or less, or may be 60% or less, but most preferably 50% or less. The type of the alcohol is not particularly limited, but examples thereof include methanol, ethanol, and propanol. By adding an alcohol or the like to the liquid, an effect of adsorbing a nucleic acid on the particles or the like is enhanced, and thus, the efficiency of extraction of a nucleic acid can be enhanced when it is used in the nucleic acid extraction device.

To the liquid, a sample containing a nucleic acid to be a target is added, whereby a nucleic acid-containing liquid 210 containing the nucleic acid is prepared. Hereinafter the nucleic acid to be a target is sometimes simply referred to as "target nucleic acid". The target nucleic acid is extracted from the sample and eluted in an eluate by the nucleic acid extraction method of this embodiment. Thereafter, for example, in the case where the nucleic acid is an mRNA, the nucleic acid is reverse-transcribed into a cDNA and is used as a template for PCR. In the case where the nucleic acid is a cDNA or a genomic DNA, the nucleic acid is directly used as a template for PCR. As the sample, blood, nasal mucus, oral mucosa, various types of biological samples, and other than these, a partially purified nucleic acid solution or the like can be used.

The nucleic acid extraction device 400 includes a tube section 440, a cup section 420 which is connected to one end of the tube section 440, and a detachable stopper 460 which seals the other end of the tube section 440.

The tube section 440 is a cylindrical part having a hollow interior portion and capable of allowing a liquid to flow through the hollow interior portion in the longitudinal direction. The tube section 440 may be bent. The size and shape of the hollow interior portion of the tube section 440 are not particularly limited as long as a liquid to be contained therein can maintain the shape of a plug in the tube section 440. Further, the size of the hollow interior portion of the tube section 440 or the shape of the cross section perpendicular to the longitudinal direction thereof may vary along the longitudinal direction of the tube section 440. Whether or not the liquid can maintain the shape of a plug in the tube section 440 depends on the conditions such as the material of the tube section 440 and the type or the like of the liquid, and therefore, the shape of the cross section perpendicular to the longitudinal direction of the tube section 440 is designed appropriately within the scope in which the liquid can maintain the shape of a plug in the tube section 440. The shape of the cross section perpendicular to the longitudinal direction of the external shape of the tube section 440 is also not limited. Further, the thickness (the length from the side surface of the hollow interior portion to the outer surface) of the tube section 440 is also not particularly limited. In the case where the shape of the cross section perpendicular to the longitudinal direction of the hollow interior portion of the tube section 440 is a circle, the inner diameter (the diameter of a circle of the cross section perpendicular to the longitudinal direction of the hollow interior portion) of the tube section 440 can be set to, for example, 0.5 mm or more and 3 mm or less. If the inner diameter of the tube section 440 is within this range, the plug composed of a liquid is easily formed in a wide range of the material of the tube section 440 and the type of the liquid, and therefore, such an inner diameter is preferred.

The material of the tube section 440 is not particularly limited, and for example, a glass, a polymer, a metal, or the like can be used. In particular, when a material transparent for visible light such as a glass or a polymer is selected as the material of the tube section 440, the interior portion (hollow interior) can be observed from the outside of the tube section 440, and therefore, such a material is preferred. Further, when a material through which a magnetic force is transmitted or a non-magnetic material is selected as the material of the tube section 440, in the case where magnetic particles are allowed to pass through the tube section 440 or the like, by applying a magnetic force from the outside of the tube section 440, the magnetic particles can be easily allowed to pass through the tube section, and thus, such a material is more preferred.

In the nucleic acid extraction method of this embodiment, a material through which a magnetic force is transmitted is selected as the material of the cup section 420 and the tube section 440, and by applying a magnetic force from the outside of the cup section 420 and the tube section 440, magnetic particles 114a are moved in the cup section 420 and the tube section 440.

The tube section 440 is internally provided, in the following order, with a first plug 441 composed of a first oil, a second plug 442 composed of a washing liquid, which is phase-separated when mixed with an oil, and with which fine particles having a nucleic acid bound thereto are washed, a third plug 443 composed of a second oil, which is immiscible with the washing liquid, a fourth plug 444 composed of an eluent, which is phase-separated when mixed with an oil, and with which the nucleic acid is eluted from the fine particles having a nucleic acid bound thereto, and a fifth plug 445 composed of a third oil, which is immiscible with the eluent.

The first plug 441, the third plug 443, and the fifth plug 445 are all composed of an oil. The oils of the first plug 441, the third plug 443, and the fifth plug 445 may be different types of oils or the same type of oil. As the oil, for example, one type of oil selected from silicone-based oils such as dimethyl silicone oil, paraffin-based oils, mineral oils, and mixtures thereof can be used. The liquids forming the adjacent plugs of the first plug 441, the second plug 442, the third plug 443, the fourth plug 444, and the fifth plug 445 are selected so that the adjacent plugs are not mixed with each other.

Between the first plug 441 and the third plug 443, the second plug 442 is disposed. In a region on the opposite side from the second plug 442 of the first plug 441, a plug composed of another liquid may be disposed. It is preferred that air bubbles or other liquids are not present in the first plug 441, however, air bubbles or other liquids may be present as long as the particles or the like having a nucleic acid adsorbed thereon can pass through the first plug 441. It is also preferred that air bubbles or other liquids are not present between the first plug 441 and the second plug 442, however, air bubbles or other liquids may be present as long as the particles or the like having a nucleic acid adsorbed thereon can pass through from the first plug 441 to the second plug 442. In the same manner, it is preferred that air bubbles or other liquids are not present between the second plug 442 and the third plug 443, however, air bubbles or other liquids may be present as long as the particles or the like having a nucleic acid adsorbed thereon can pass through from the second plug 442 to the third plug 443.

Between the third plug 443 and the fifth plug 445, the fourth plug 444 is disposed. In a region on the opposite side from the fourth plug 444 of the fifth plug 445, a plug composed of another liquid may be disposed. It is preferred that air bubbles or other liquids are not present in the third plug 443, however, air bubbles or other liquids may be present as long as the particles or the like having a nucleic acid adsorbed thereon can pass through the third plug 443. It is also preferred that air bubbles or other liquids are not present between the third plug 443 and the fourth plug 444, however, air bubbles or other liquids may be present as long as the particles or the like having a nucleic acid adsorbed thereon can pass through from the third plug 443 to the fourth plug 444. In the same manner, it is preferred that air bubbles or other liquids are not present between the fourth plug 444 and the fifth plug 445, however, air bubbles or other liquids may be present as long as the particles or the like having a nucleic acid adsorbed thereon can pass through from the fourth plug 444 to the fifth plug 445. Further, it is preferred that air bubbles or other liquids are not present in the fifth plug 445.

The length of each of the first plug 441, the third plug 443, and the fifth plug 445 in the longitudinal direction of the tube section 440 is not particularly limited as long as it is within a range capable of forming the plug. A specific length of each of the first plug 441, the third plug 443, and the fifth plug 445 in the longitudinal direction of the tube section 440 is 1 mm or more and 50 mm or less, and preferably 1 mm or more and 30 mm or less, more preferably 5 mm or more and 20 mm or less so that the moving distance of the particles or the like is not too long. When the length in the longitudinal direction of the tube section 440 of the third plug 443 among these plugs is made longer, in the case of adopting a configuration in which the fourth plug 444 is ejected from the end of the tube section 440 on the fifth plug 445 side, this can make it difficult to eject the second plug 442. In this case, a specific length of the third plug 443 can be set to 10 mm or more and 50 mm or less.

The first plug 441 and the fifth plug 445 each have a function of preventing substance exchange with the outside air such as evaporation or contamination from the outside of the washing liquid (the second plug 442) and the eluent (the fourth plug 444) even if at least one end of the tube section 440 is opened. Therefore, even if at least one end of the tube section 440 is opened to the outside air, the volume of the washing liquid or the eluent can be maintained constant, and a variation in concentration of each liquid or contamination thereof can be prevented. Due to this, the accuracy of concentration of a nucleic acid or each agent in nucleic acid extraction can be enhanced.

The third plug 443 has a function of preventing mixing of the washing liquid (the second plug 442) and the eluent (the fourth plug 444) with each other. Further, by using an oil having a higher viscosity as the third plug 443, a "wipe-off effect" of the oil at the interface between the third plug 443 and the washing liquid (the second plug 442) can be enhanced when the particles or the like are moved. Accordingly, when the particles or the like are moved to the third plug 443 composed of the oil from the plug composed of the washing liquid (the second plug 442), it can make it more difficult to carry over water-soluble components adhered to the particles or the like into the third plug 443 (oil).

The second plug 442 is disposed at a position between the first plug 441 and the third plug 443 in the tube section 440. The second plug 442 is composed of the washing liquid for washing fine particles having a nucleic acid bound thereto. The washing liquid is a liquid immiscible with both of the oil constituting the first plug 441 and the oil constituting the third plug 443.

The washing liquid is an acidic solution, and is particularly preferably an acidic aqueous solution. The acid to be contained is not particularly limited, however, an aqueous solution of citric acid, acetic acid, glycine hydrochloride, or the like is preferred. The washing liquid may contain EDTA (ethylenediaminetetraacetic acid), a surfactant (such as Triton, Tween, or SDS), or the like, but is preferably a solution which does not substantially contain chaotropic substances. The pH of the solution is not particularly limited as long as the washing solution is acidic, but the lower limit of the pH is preferably 1 or more, more preferably 2 or more, further more preferably 3 or more, and most preferably 4 or more. The upper limit of the pH is preferably 6 or less, more preferably 5 or less, and most preferably 4 or less. By adopting such a washing liquid, even if a nucleic acid or a particle having a nucleic acid bound thereto comes in contact with a solution containing an alcohol on the upstream side of the first plug, that is, even in the case where a nucleic acid is extracted with an adsorbing liquid containing an alcohol, which will be described below, or the case where particles having a nucleic acid adsorbed thereon are washed with a washing liquid containing an alcohol, the particles or the like having a nucleic acid adsorbed thereon can be efficiently washed with the washing liquid, and also an alcohol can be prevented from being carried downstream, that is, so-called carry-over of an alcohol can be prevented.

The volume of the second plug 442 is not particularly limited, and can be appropriately set by using the amount of the particles or the like having a nucleic acid adsorbed thereon or the like as an index. For example, when the volume of the particles or the like is 0.5 µL, it is sufficient that the volume of the second plug 442 is 10 µL or more, and it is set to preferably 20 µL or more and 50 µL or less, more preferably 20 µL or more and 30 µL or less. If the volume of the second plug 442 is within this range, when the volume of the particles or the like is 0.5 µL, the particles or the like can be sufficiently washed. The volume of the second plug 442 is preferably larger for washing the particles or the like, but can be appropriately set in consideration of the length or diameter of the tube section 440, the length or the like of the second plug 442 in the longitudinal direction of the tube section 440 depending thereon.

The fourth plug 444 is disposed at a position between the third plug 443 and the fifth plug 445 in the tube section 440 and is composed of an eluent with which a nucleic acid is eluted from fine particles having a nucleic acid bound thereto.

As the eluent, for example, purified water such as sterile water, distilled water, or ion exchanged water, or a buffer can be used. When water or an aqueous solution is used as the eluent, by dipping the particles or the like having a nucleic acid adsorbed thereon in an eluent, the nucleic acid adsorbed on the particles or the like can be eluted. The eluent is a liquid immiscible with both of the oil constituting the third plug 443 and the oil constituting the fifth plug 445.

The volume of the fourth plug 444 composed of the eluent is not particularly limited, and can be appropriately set by using the amount of the particles or the like having a nucleic acid adsorbed thereon or the like as an index. For example, when the volume of the particles or the like is 0.5 µL, it is sufficient that the volume of the fourth plug 444 composed of the eluent is 0.5 µL or more, and it is set to preferably 0.8 µL or more and 5 µL or less, more preferably 1 µL or more and 3 µL or less. If the volume of the plug composed of the eluent is within this range, even when the volume of the fine particles is set to 0.5 µL, the nucleic acid can be sufficiently eluted from the fine particles.

The material of the stopper 460 may be any as long as it is a material capable of detachably sealing the other end of the tube section 440, and the stopper 460 is formed from, for example, a rubber, an elastomer, a polymer, or the like. The stopper 460 may be in contact with the fifth plug 445 when it seals the end, or a gas such as air may be disposed between the fifth plug 445 and the stopper 460. The stopper 460 is freely detachable, but its mechanism is not particularly limited.

The cup section 420 has a shape capable of pouring the liquid ejected from the outlet port 110 of the vessel 100 in the cup section 420. The material of the cup section 420 is a material having flexibility such as a rubber, an elastomer, or a polymer. As described above, by closing the opening section of the cup section 420 with the cap 470 and deforming the cup section 420, pressure can be applied to the inside of the tube section 440 of the nucleic acid extraction device 400. By doing this, it is easy to apply pressure to the tube section 440 from the connection side of the vessel 100 for ejecting the eluate (fourth plug 444) from the end of the tube section 440. Accordingly, the eluate can be dispensed in, for example, a reaction vessel or the like for PCR.

Next, the respective steps of the nucleic acid extraction method will be described.

First, the sample is introduced into the bottle 200, whereby the nucleic acid-containing liquid 210 is prepared (FIG. 4A). The preparation of the nucleic acid-containing liquid 210 can be performed by adhering the sample to a cotton swab 300, inserting the cotton swab 300 from the opening section 220 of the bottle 200 and dipping the cotton swab 300 in the liquid in the bottle 200. The sample may also be introduced from the opening section 220 of the bottle 200 using a pipette or the like. In the case where the sample is in the form of a paste or a solid, for example, the sample may be adhered to the inner wall of the bottle 200 or thrown into the bottle 200 from the opening section 220 of the bottle 200 using a spoon, forceps, or the like.

Subsequently, the opening section 106 of the vessel 100 according to the invention and the opening section 220 of the bottle 200 are fitted together, whereby the vessel 100 and the bottle 200 are integrated (FIG. 4B).

Subsequently, a nucleic acid in the liquid 210 is bound to the magnetic particles (FIG. 4C). By turning upside down the integrated body of the vessel 100 and the bottle 200, the nucleic acid-containing liquid 210 is sent into the vessel 100 from the inside of the bottle 200. In the vessel 100, the nucleic acid-containing liquid passing through the coarse-mesh filter passes through the fine-mesh filter. The nucleic acid-containing liquid passing through the fine-mesh filter comes in contact with the lyophilizate disposed on a bottom portion of the vessel 100. At this time, the magnetic particles in the lyophilizate are mixed in the nucleic acid-containing liquid. The target nucleic acid is adsorbed on the surfaces of the magnetic particles by the action of a chaotropic agent and bound thereto. Here, nucleic acids other than the target nucleic acid, or proteins passing through the filter may be adsorbed on the surfaces of the magnetic particles.

It is not necessary to shake the vessel using a device such as a vortex shaker, or to perform mixing by shaking the vessel with the hand of an operator for binding the target nucleic acid to the surfaces of the magnetic particles in this manner. Due to this, according to the invention, the nucleic acid can be bound to the nucleic acid-binding solid-phase carrier in a shorter time by omitting an operation for dispersing the nucleic acid-binding solid-phase carrier.

Subsequently, the nucleic acid bound to the magnetic particles is eluted in an eluate in the tube section 440 (FIG. 4D). As the method of introducing the magnetic particles having the nucleic acid adsorbed thereon into the tube section 440, a method utilizing a gravitational force or a centrifugal force may be used, and there is no particular limitation on the method, however, in this embodiment, a method in which a magnetic force is applied using a permanent magnet 480 from the outside of the cup section 420 and the tube section 440 is used. A magnetic force can be applied using, for example, an electromagnet, or the like other than the permanent magnet, however, from the viewpoint that heat is not generated and so on, it is more preferred to apply a magnetic force using a permanent magnet. In the case of using a permanent magnet, the magnet may be moved by an operator with hand or by using a mechanical device or the like. The magnetic particles have a property to be attracted by a magnetic force, and therefore, by utilizing the property, the magnetic particles are moved from the inside of the cup section 420 to the tube section 440 by changing the relative position of the permanent magnet to the cup section 420 and the tube section 440. In this embodiment, the magnetic particles are moved by moving the permanent magnet 480 in the direction indicated by the arrow in FIG. 4D. By doing this, the magnetic particles are moved from the first plug 441 to the fourth plug 444 by sequentially passing the particles through the respective plugs. The retention time of the magnetic particles in each plug when the magnetic particles pass through the plug is not particularly limited, and also the magnetic particles may be moved reciprocatively along the longitudinal direction of the tube section 440 within the same plug.

When the magnetic particles reach the fourth plug 444, the nucleic acid adsorbed on the magnetic particles is eluted in an eluate in the fourth plug 444 by the action of the eluent. By undergoing this step, the nucleic acid is eluted with the eluent from the sample, and the nucleic acid is brought to a state of being extracted from the sample.

Subsequently, the eluate in the fourth plug 444 is ejected from the tube section 440 (FIG. 4E). In this embodiment, the cap 470 is attached to the cup section 420, and the stopper 460 is detached from the tube section 440 of the nucleic acid extraction device 400 to open the end of the tube section 440 on the fifth plug 445 side. Then, the cup section 420 is deformed by applying an external force to the cup section 420 in the direction indicated by the arrows in FIG. 4E to increase the internal pressure of the tube section 440, the respective plugs in the tube section 440 are moved from the first plug 441 side to the fifth plug 445 side due to the pressure. Accordingly, the fifth plug 445 and the fourth plug 444 are ejected in this order from the end of the tube section 440 on the fifth plug 445 side.

As described above, according to the vessel 100 of this embodiment, the time and labor required for dispersing the nucleic acid-binding carrier in the pretreatment for preparing a nucleic acid for use in PCR or the like in the related art can be reduced.

### Protein Purification Method

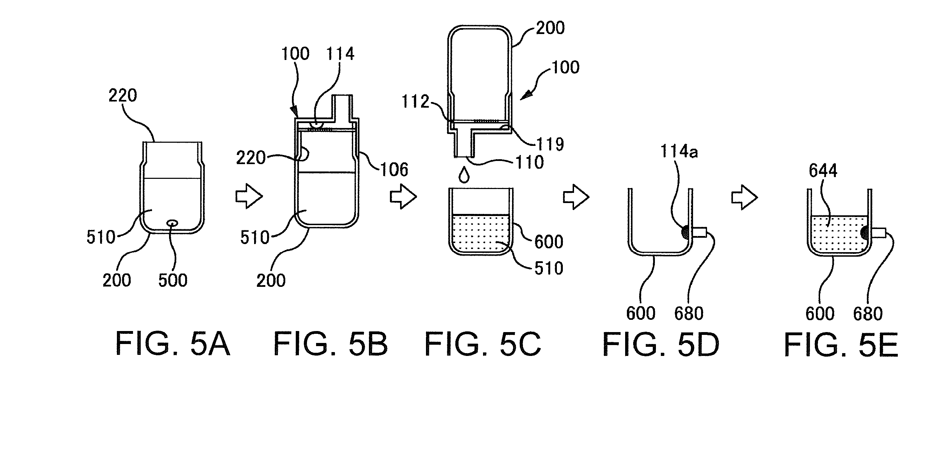

Next, a method of purifying a protein from cells using the vessel 100 according to this embodiment will be described with reference to FIGS. 5A to 5E. FIGS. 5A to 5E are schematic views showing a protein purification method using the vessel according to the embodiment.

A vessel 100 and a bottle 200 to be used in the protein purification method are configured such that an upper portion of the bottle 200 has an opening section 220 in the same manner as that used in the nucleic acid extraction method. In this embodiment, the opening section 220 of the bottle 200 is configured to be capable of being fitted with an opening section 106 of the vessel 100, and by fitting the opening sections together, the inside of the bottle 200 and the inside of the vessel 100 can communicate with each other. In a lyophilizate 114 in the vessel 100, magnetic particles surface-modified with a substance having affinity for a protein in cells to be a target for purification (hereinafter referred to as "target protein"), for example, protein A, protein G, biotin, avidin, glutathione, lectin, or an antibody are contained. The substance bound to the magnetic particles can specifically bind to a protein having an activity to be specifically adsorbed on the substance.

In order to purify a protein from cells, first, a lysis buffer 510 is placed in the bottle 200 (FIG. 5A). The lysis buffer 510 may be any as long as it is a liquid capable of lysing cells without denaturing the target protein. For example, a liquid containing Tris-HCl, EDTA, EGTA, SDS, a deoxycholate, and Triton-X or NP-40 can be used after the pH of the liquid is adjusted to be most suitable for obtaining the target protein. Then, cells 500 are introduced into the lysis buffer 510 in the bottle 200, whereby a protein-containing liquid 510 is prepared.

Subsequently, the opening section 106 of the vessel 100 according to the invention and the opening section 220 of the bottle 200 are fitted together to integrate the vessel 100 with the bottle 200 (FIG. 5B).

Subsequently, the protein in the lysis buffer 510 is bound to the substance having affinity for the protein on the substances of the magnetic particles (FIG. 5C). Then, the integrated body of the vessel 100 and the bottle 200 is turned upside down, whereby the protein-containing liquid 510 is sent into the vessel 100 from the bottle 200. In the vessel 100, the protein-containing liquid passing through the coarse-mesh filter passes through the fine-mesh filter. The protein-containing liquid passing through the fine-mesh filter comes in contact with the lyophilizate 114 disposed on a bottom portion of the vessel 100. At this time, the magnetic particles in the lyophilizate are mixed in the protein-containing liquid. The target protein is bound to the substance having affinity for the protein on the surfaces of the magnetic particles. Here, proteins other than the target protein passing through the filter may be adsorbed on the surfaces of the magnetic particles. The protein-containing liquid flowing out from the outlet port 110 of the vessel 100 is allowed to flow in a vessel 600.

It is not necessary to shake the vessel using a device such as a vortex shaker, or to perform mixing by shaking the vessel with the hand of an operator for binding the target protein to the surfaces of the magnetic particles in this manner. Due to this, according to the invention, the protein can be bound to the protein-binding solid-phase carrier in a shorter time by omitting an operation for dispersing the protein-binding solid-phase carrier.

Subsequently, a permanent magnet 680 is brought closer to the outer surface of the vessel 600, and by the magnetic force thereof, the liquid is discharged to the outside of the vessel 600 while keeping the magnetic particles on the side wall of the vessel 600 (FIG. 5D).

Subsequently, by adding an appropriate eluent 644 to the vessel 600, the protein is eluted from the magnetic particles (FIG. 5E). After the elution, the liquid which is the supernatant is collected from the vessel 600 while keeping the magnetic particles in the vicinity of the side wall of the vessel 600 by bringing the permanent magnet 680 closer to the side wall of the vessel 600 from the outside, whereby the protein bound to the magnetic particles can be recovered. As the eluent for eluting the protein, a liquid having an effect of decreasing the affinity between the protein and the ligand is preferred. Examples of the liquid include a buffer containing hydrochloric acid, imidazole, glutathione, and biotin. Examples of the buffer include potassium phosphate buffer, sodium phosphate buffer, Tris-HCl buffer, PIPES buffer, boric acid buffer, and glycine hydrochloride buffer.

The magnetic particles may be washed before the protein is eluted from the magnetic particles to remove contaminants. Specific examples of the washing method include a method in which redispersion of the magnetic particles is repeated by adding the magnetic particles having the protein bound thereto to sodium phosphate buffer containing Tween 20 (0.5% by mass) to remove contaminants adsorbed on the magnetic fine particles. As described above, according to the vessel 100 of this embodiment, the time and labor required for dispersing the protein-binding carrier in the pretreatment for preparing a protein in the related art can be reduced.

The invention is not limited to the above-described embodiments, and further, various modifications can be made. For example, the invention includes substantially the same configurations (for example, configurations having the same functions, methods, and results, or configurations having the same objects and effects) as the configurations described in the embodiments. In addition, the invention includes configurations in which parts which are not essential in the configurations described in the embodiments are substituted. In addition, the invention includes configurations that exhibit the same operations and effects as those of the configurations described in the embodiments, or configurations that can achieve the same objects. In addition, the invention includes configurations in which well-known techniques are added to the configurations described in the embodiments.

## Claims

1. A lyophilizate comprising (i) a solid-phase carrier which can bind a protein or a nucleic acid and (ii) an additive which keeps the carrier in a dispersed state..

2. The lyophilizate according to claim 1, wherein the solid-phase carrier is a magnetic particle.

3. The lyophilizate according to claim 1 or claim 2, wherein
the additive is at least one member selected from trehalose, sucrose, polyvinylpyrrolidone, carboxymethyl cellulose, dextrin, dextran, polyethylene glycol, cyclodextrin, and maltodextrin.

4. A vessel for binding a protein or nucleic acid comprised in a liquid to a solid-phase carrier, wherein
a lyophilizate according to any preceding claim is provided in the vessel.

5. The vessel according to claim 4, wherein the vessel comprises:
an opening section for introducing the substance-containing liquid;
a filter section for filtering the substance-containing liquid introduced from the opening section;
a contact section for bringing the substance-containing liquid passing through the filter section and the lyophilizate into contact with each other; and
an outflow section for allowing the substance-containing liquid brought into contact with the lyophilizate in the contact section to flow out to the outside of the vessel.

6. A method of producing a lyophilizate according to any of claims 1-3, the method comprising lyophilizing the solid-phase carrier and the additive.
